# NEW EUROPEAN PATENT SPECIFICATION

(11) **EP 0 407 028 B2**
(45) Date of publication and mention of the opposition decision: **07.07.1999**
(45) Mention of the grant of the patent: 14.09.1994
(21) Application number: 90305810.5
(22) Date of filing: 29.05.1990
(51) Int. Cl.: A61M 15/00

(54) **Medicament inhalation device and formulation**
Medikament und Inhalationsvorrichtung dafür
Formule médicamenteuse et appareil d'inhalation

(30) Priority: 31.05.1989 GB 8912503; 10.06.1989 GB 8913392
(43) Date of publication of application: 09.01.1991
(73) Proprietor: FISONS plc, West Malling, Kent ME19 4TA (GB)
(72) Inventor: Clark, Andrew Reginald, Loughborough, Leicestershire (GB); Hart, John Leck, Bramcote, Nottingham NG9 3BB (GB); Drought, Nicholas, Andrew, Murray,, Cambridge CB4 6DS (GB)
(74) Representative: Caffin, Lee

(56) References cited:
- WO-A-93/24165
- FR-A- 2 352 556
- FR-A- 2 516 387
- GB-A- 1 118 341
- GB-A- 1 569 611
- GB-A- 2 179 260
- SE-A- 8 104 240
- US-A- 2 587 215
- US-A- 2 622 594
- US-A- 4 534 345

## Description

This invention relates to a device for the administration of powdered medicaments by inhalation, more particularly to a multiple-dose device with metering means for the dispensing of doses from a medicament reservoir, and also to medicament formulations for use therein.

The administration by inhalation of medicaments in dry powder form is well known. Devices for the metering and dispensing of measured doses of medicament from a reservoir have also been described previously (for example in UK Patent No 2041763 and US-A-2587215). Such devices typically comprise a medicament reservoir and a metering chamber with a volume chosen such that, when filled, the chamber contains the desired weight of medicament for one dose. Filling of the metering chamber is generally accomplished under the influence of gravity, the chamber typically being located at the bottom of the reservoir. Such devices have the disadvantage that variations in the density of the metered powder can easily occur resulting in Inaccurate or inconsistent dosing. The packing density of the powder may also depend on the weight of powder remaining in the reservoir, leading to a gradual reduction in the dose delivered by the device. In addition, the dose metered is strongly dependent on the orientation of the device.

Furthermore, when the medicament to be administered is hygroscopic, great care must be taken to ensure that water does not contaminate the medicament because this may cause lumps of medicament to form which can clog the device and lead to inconsistent dosing. The problem of water uptake by hygroscopic medicaments is overcome to some extent by supplying them in gelatin capsules which are punctured immediately prior to administration in a suitable device (for example the device of UK Patent 1122284). However, a fresh capsule must be inserted into the device for each dose, and the volume occupied by a month's supply of capsules is considerable. In addition, such devices have the further disadvantage that their performance is dependent upon the relative humidity of the atmosphere in which they are used, ie the quality of cloud (the proportion of particles in the cloud which are fine enough to penetrate deep into the lung) decreases as the relative humidity increases. Furthermore, if the gelatin capsules are stored in an atmosphere of high relative humidity, they become soft and consequently difficult to handle.

FR-A-2516387 discloses a device wherein a volatile medicament is released into the air to be inhaled by breaking microcapsules containing the medicament into the air stream.

GB-A-2179260 discloses a device comprising a body defining an endless orbital path communicating with an air inlet and an air outlet. A ball provided within the orbital path travels around the path upon inhalation by a patient, thus releasing medicament coated on the ball or on the inner surface of the path into the inhaled airstream. The device has the disadvantages that the orbital path is difficult to manufacture which consequently makes the device expensive, and that some medicaments are not delivered satisfactorily from it, notably those which have a unit dose of comparatively large mass. In addition, there is a danger that the dose delivered to a patient from such a device may vary widely since it will depend upon attrition of the coating by impaction of the ball on the surface. The dose delivered may therefore depend, amongst other things, upon the force with which air is inhaled through the device.

US 2,622,594 discloses a snuff dispenser in which snuff is scraped of the surface of a body of snuff contained in the device, however, there is no metering means for predetermining the amount of snuff which is dispensed, the dose being dependent on the whim of the user.

B.M. Wright in J. Sci. Instrum., 1950, 27, 12, describes a device for generating clouds of e.g. coal dust or silica dust, wherein dust is scraped of the surface of a cake of compressed dust. The device suffers from the problem that the dust tends to be scraped off in large aggregates which must be passed through a jet-impactor to obtain adequate dispersion. Furthermore, the dust is produced continuously rather than as a predetermined dose.

We have now found that these disadvantages, can be overcome or substantially mitigated by the use of a metering means which relies not on gravitational force to fill a metering chamber, but on abrasion of a compacted body of powdered medicament.

Thus, according to a first aspect of the present invention, there is provided a device for the administration by inhalation of a medicament in powdered form as defined in claim 1.

The devices of the present invention may be designed for limited use (ie supplied with an integral compacted body of medicament and discarded when that is exhausted), or to be reusable (ie replacement compacted bodies may be inserted).

Thus according to a further aspect of the invention we provide a device for the administration by inhalation of a medicament in powdered form, as defined in Claim 2.

By "compacted body of powdered medicament" we mean a body of medicament produced by compressing a sample of loose powder so that the medicament particles hold together. The degree of compaction obtained will clearly depend upon the compression force applied to the sample of loose powder, and the term "compacted body of powdered medicament" includes compacted bodies of medicament ranging from those that are loosely compacted to those that are tightly compacted.

A loosely compacted body of medicament may be obtained by applying a pressure of, for example, from 1x10⁴ to 15x10⁴Nm⁻² to a sample of loose powder (for example a compression force as might be applied by biassing means in certain embodiments of the first aspect of the present invention), and the degree of compaction would be insufficient for such bodies to retain their structural integrity upon handling.

A tightly compacted body of medicament may be obtained by applying a pressure of, for example, from 30x10⁴ to 150x10⁴Nm⁻² to a sample of loose powder (for example a compression force as might be applied by a small hydraulic press), and the degree of compaction would be sufficient for such bodies to retain their structural integrity upon handling.

The medicament may consist solely of an active ingredient, for example a hygroscopic drug. Active ingredients which may be mentioned include sodium cromoglycate, nedocromil sodium. salbutamol and terbutaline. We prefer the medicament to additionally comprise an inert carrier, especially in the case of tightly compacted bodies of medicament, because this results in improved compaction and dispersion characteristics. Suitable inert carriers include sugars, for example lactose. When an inert carrier is present we prefer the particle size of the carrier to be larger than that of the active ingredient. Suitable particle sizes for the active ingredient when in loose powder form are from 1 to 10µm.

Formulations of the medicament which may be mentioned include a mixture of any one of the above-mentioned active ingredients in association with lactose, the proportions of carrier to active ingredient depending upon the particular substances present. For example, we have found that a 1:1 mixture of nedocromil sodium or sodium cromoglycate with lactose is advantageous.

Tightly compacted bodies of powdered inhalation medicament are novel. Thus, according to a further aspect of the present invention, there is provided a compacted body of powdered inhalation medicament for administration from an inhalation device as defined in Claim 11.

This further aspect of the invention also includes compacted bodies of powdered inhalation medicament comprising more than one active ingredient, and compacted bodies comprising more than one inert carrier.

The compacted body of inhalation medicament may have any convenient external shape, for example cylindrical or brick-like, and in the case of a loosely compacted body will generally be determined by the inner configuration of the medicament reservoir.

Desirably, the means for abrading includes a blade which cuts, scrapes or otherwise erodes a surface of the compacted body by relative rotation or sliding between them. For example, the blade may have a helical shape, in which case the dose abraded from the compacted body of medicament will depend upon the pitch of the helix, the diameter of the blade, the density of the compacted body, and the angle through which the blade is rotated. Thus, when the device is provided with a helical blade, we prefer it to be further provided with blade rotation control means, for example a ratchet which will only permit rotation through a predetermined angle for each dose.

The compacted body may be moved towards the abrading means by a predetermined distance to permit the correct amount of medicament to be removed for one dose. This movement may be achieved by screw means (for example a mechanism similar to that used in lipsticks or glue in stick form) or by biassing means such as a spring urging the compacted body towards the abrading means. Alternatively, the compacted body may be fixed and the abrading means may move towards it.

The inner surfaces of devices according to the invention which are in contact with the compacted body of medicament (for example the abrading means and the interior of the reservoir) may advantageously be coated with a friction reducing agent, for example PTFE (polytetrafluoroethene).

We prefer devices according to the invention to include a through-going pathway connecting an air inlet with an air outlet, for example a mouthpiece such that, in use, a metered dose of medicament is deposited in the through-going pathway and is then inhaled by a patient inhaling at the air outlet.

We have also found a particular arrangement of air passageways (referred to herein as 'cyclone means') which is especially useful in inhalation devices according to the present invention. Such cyclone means have been found to help improve the quality of the medicament cloud delivered to a patient. Thus, devices according to the invention may include a through-going pathway including a chamber within which entrained medicament may circulate, wherein the inlet from the pathway to the chamber is tangential to the chamber wall and orthogonal to the longitudinal axis of the chamber, the outlet from the chamber is situated on the longitudinal axis of the chamber so that entrained medicament is preferentially removed through the outlet from the central zone of the chamber, and the inlet passageway includes a venturi adjacent to the junction of the inlet passageway with the chamber.

When cyclone means are used in association with devices according to the present invention, the metered dose is preferably deposited in the through-going pathway upstream from the inlet to the chamber. A patient then inhales through a mouthpiece communicating with the outlet from the chamber and thus inhales the medicament which is entrained in the resulting airstream. Such cyclone means have the advantage that the finer particles in a particle population are selected for inhalation. They also cause the bolus of entrained powder to be spread out more evenly so that the dose of medicament is inhaled over a longer period of time.

Other means for improving the quality of the medicament cloud which may be provided in the through-going pathway include grids, propellers and vanes through which air and entrained medicament pass.

The devices of the present invention overcome the disadvantages of prior art devices in that a month's supply of medicament occupies a much smaller volume than, for example, an equivalent number of gelatin capsules. In addition, we have surprisingly found that the quality of the medicament cloud produced by devices of the present invention charged with compacted bedies of medicament according to the second aspect of the invention is not affected by the relative humidity of the atmosphere in which they are used, the performance remaining comparable with, and in some cases superior to the device of UK Patent 1122284 when it is operated in an atmosphere of low relative humidity. We have also unexpectedly found that such devices may be stored in an atmosphere of high relative humidity without adversely affecting the quality of the medicament cloud eventually obtained from them.

Further, the devices of the present invention have the advantage that they are very simple to use, in some embodiments a twist of the medicament-compact holding chamber being all that is required before inhaling through the mouthpiece. Also, the dosing consistency is much greater than is found with devices in which filling of a metering chamber is accomplished under the influence of gravity (as in the device of UK Patent No 2041763). In addition they do not have the disadvantage that actuation of the device must be synchronised with inhalation as with pressurized aerosol devices.

Although the invention has been described for use in oral inhalation of medicaments, it is also suitable for the administration of nasal medicaments by inhalation. The necessary adaptation for this mode of administration will be readily apparent to those skilled in the art.

Preferred embodiments of the present invention will now be described, by way of example, with reference to the following drawings, in which corresponding features are given the same reference numeral:
Figure 1 is a perspective view of an inhalation device according to the invention;
Figure 2 is vertical cross sectional view of the device of Figure 1;
Figure 3 is a perspective view of the medicament reservoir and metering means of the device of Figure 1, partly cut away;
Figure 4 is an alternative embodiment of a device according to the invention;
Figure 5 is a schematic view of an arrangement of air passageways for use in a device according to the invention;
Figure 6 is a lateral cross sectional view of the arrangement of Figure 5 in the plane VI-VI-VI; and
Figure 7 is a vertical cross sectional view of a device according to the invention incorporating the arrangement of air passageways shown in Figures 5 and 6.

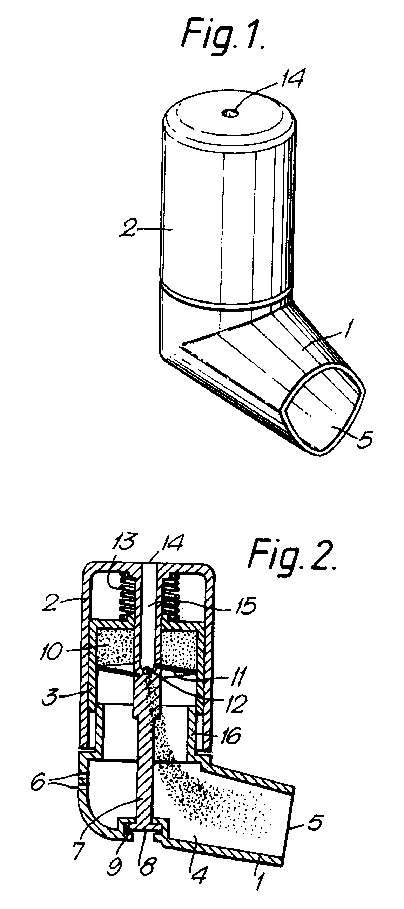

Referring first to Figures 1 and 2, a device according to the invention comprises a lower body portion 1, and an upper body portion 2 which encloses a medicament reservoir 3.

Lower body portion 1 defines a tubular chamber 4 having an opening 5 in a mouthpiece portion. Air inlets 6 are provided in the wall of lower body portion 1 opposite opening 5.

Upper body portion 2 is generally cylindrical and is rotatably mounted on lower body portion 1 over a second opening therein by an axial shaft portion 7 having a flanged end 8 which is received by a socket 9 provided in the lower wall of lower body portion 1.

Medicament reservoir 3 is also generally cylindrical, and is slidably mounted in upper body portion 2. Shaft 7 passes axially through reservoir 3 and also through an axial bore formed in a cylindrical block of tightly compacted medicament 10 which is situated in reservoir 3.

A helical blade 11 is attached to shaft 7 towards the middle of its length and extends radially to the inner wall of reservoir 3. The edge of blade 11 describes an arc of approximately 350°, and a further air inlet 12 is positioned between the leading and trailing edges of blade 11. Air inlet 12 communicates with an external opening 14 situated in the centre of the upper face of upper body portion 2 via passageway 15 which runs axially through shaft 7.

The block of compacted medicament 10 is urged towards blade 11 by spring 13 which acts against the inner wall of upper body portion 2 and the outer wall of reservoir 3.

To use the device, upper body portion 2 is rotated through 45° (further rotation being prevented by a ratchet mechanism which is omitted for clarity) which results in blade 11 cutting into block of medicament 10 and abrading the quantity of medicament required for one dose. Some of the abraded medicament is deposited in lower body portion 1, and some remains between the leading and trailing edges of blade 11. A patient then inhales through opening 5, causing air to be drawn in through nets 6 and opening 14, resulting respectively in medicament being entrained from lower body portion 1 and from between the leading and trailing edges of blade 11. The entrained medicament is then inhaled by the patient.

It will be appreciated that upper body portion 1 is stabilized by the wall of medicament reservoir 3 sliding telescopically over a peripheral wall 16 formed around the second opening in lower body portion 1 as block of medicament 10 is abraded. If necessary, cooperating vertical tongues and grooves may be provided on the inner and outer faces of the wall of medicament reservoir 3 and peripheral wall 16 in order to prevent relative rotation between them, which would reduce the effectiveness of blade 11.

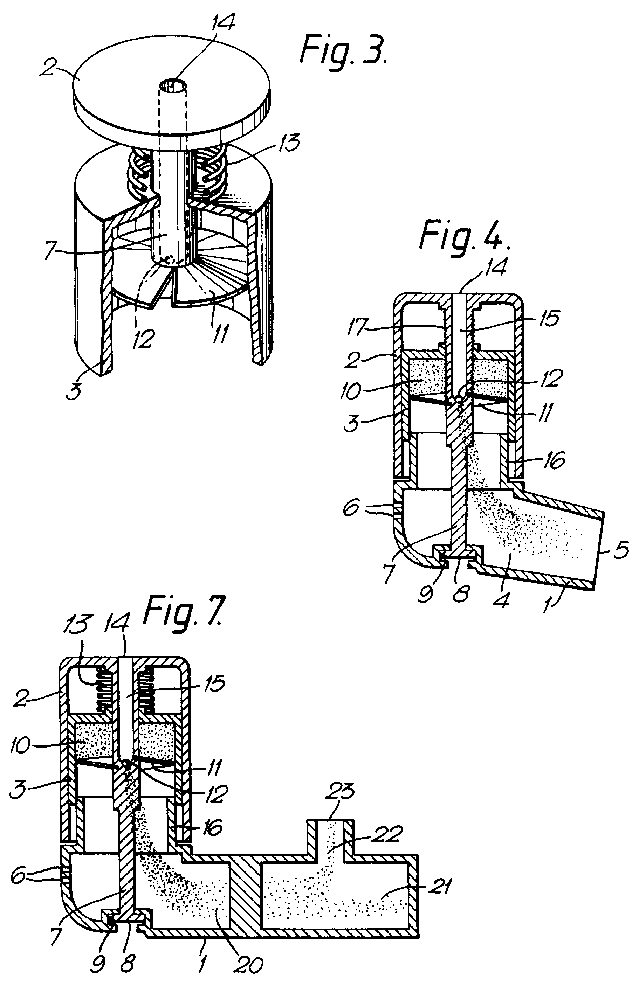

Referring now to Figure 4, an alternative embodiment resembles that of Figure 1, except that medicament reservoir 3 is moved towards blade 11 by the action of a screw thread 17 formed on the upper portion of shaft 7 upon which the upper opening of medicament reservoir 3 is threaded. The length of block 10 abraded by blade 11 as it turns through a certain angle coincides with the distance which medicament reservoir 3 is moved down shaft 7 by the action of screw thread 17.

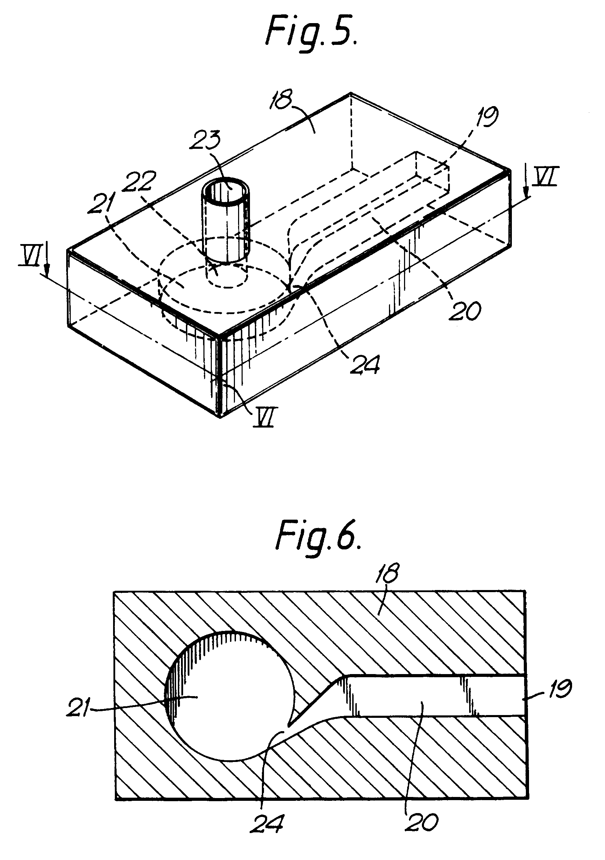

Referring now to Figures 5 and 6, an arrangement of air passageways for use in a device according to the invention comprises a body 18 provided with an air inlet 19 which communicates via an inlet passageway 20 with a cylindrical chamber 21 into which the inlet passageway 20 empties tangentially; an outlet passageway 22 situated on the longitudinal axis of the chamber, the diameter of the outlet passageway's opening being about one third that of the chamber; and a mouthpiece 23 situated at the end of the outlet passageway. Inlet passageway 20 is orthogonal to outlet passageway 22. Adjacent to the junction of inlet passageway 20 with chamber 21, there is a local constriction (venturi) 24 in the inlet passageway.

To use the device illustrated in the drawings, a dose of medicament is deposited in inlet passageway 20 (by means not shown) adjacent to air inlet 19. A patient then inhales through mouthpiece 23 which causes air to be drawn into inlet passageway 20. The inhaled stream of air entrains the medicament, which then passes into cylindrical chamber 21 from which it leaves through outlet passageway 22 and is then delivered to the patient's mouth through mouthpiece 23.

Referring now to Figure 7, an inhalation device comprises the features of the device shown in Figure 1 with the arrangement of air passageways shown in Figure 5 being incorporated into lower body portion 1. In use, a dose of medicament is abraded from block of medicament 10 and deposited in inlet passageway 20. A patient then inhales at mouthpiece 23.

## Claims

1. A device for the administration by inhalation of a medicament in powdered form, comprising a medicament reservoir (3) adapted to receive a compacted body of powdered medicament (10) and metering means (11) for dispensing medicament from the reservoir (3), the metering means including means for abrading the compacted body (10), characterized in that biassing means or screw means is provided for moving the compacted medicament body and abrading means towards one another to permit the correct amount of medicament to be abraded from the compacted medicament body for one dose.

2. A device for the administration by inhalation of a medicament in powdered form, comprising a medicament reservoir (3) comprising a compacted body of powdered medicament (10) and metering means (11) for dispensing medicament from the reservoir (3), the metering means including means for abrading the compacted body (10), characterized in that biassing means or screw means is provided for moving the compacted medicament body and abrading means towards one another to permit the correct amount of medicament to be abraded from the compacted medicament body for one dose.

3. A device according to claim 1 or 2, wherein the means for abrading includes a blade (11).

4. A device according to any one of the preceding claims, wherein the compacted body (10) is urged towards the means for abrading (11) by biassing means (13).

5. A device according to any one of the preceding claims, wherein the compacted body of powdered medicament (10) is loosely compacted.

6. A device according to any one of claims 1 to 4, wherein the compacted body of powdered medicament (10) is tightly compacted, the degree of compaction being sufficient to impart the body with structural integrity upon handling.

7. A device according to any one of the preceding claims, wherein the active ingredient of the medicament is hygroscopic.

8. A device according to any one of the preceding claims, wherein the active ingredient of the medicament is selected from sodium cromoglycate and nedocromil sodium.

9. A device according to any one of the preceding claims, wherein the medicament includes an inert carrier.

10. A device according to any one of the preceding claims, wherein a metered dose dispensed from the reservoir (3) is deposited in a through-going pathway including a chamber (21) within which entrained medicament may circulate, wherein the inlet (20) from the pathway to the chamber (21) is tangential to the chamber wall and orthogonal to the longitudinal axis of the chamber, and the outlet (23) from the chamber (21) is situated on the longitudinal axis of the chamber so that entrained medicament is preferentially removed through the outlet (23) from the central zone of the chamber (21), the inlet passageway (20) including a venturi (24) adjacent to the junction of the inlet passageway with the chamber (21).

11. A compacted body of powdered inhalation medicament for administration from an inhalation device comprising a medicament reservoir (3) adapted to receive said compacted body of powdered medicament (10) and metering means (11) for dispensing medicament from the reservoir (3), the metering means including means for abrading the compacted body (10); characterised in that the body comprises a loose powder including an active ingredient having a particle size of from 1 to 10 µm compressed to form a tightly compacted body, the degree of compaction being sufficient to impart the body with structural integrity upon handling such that a plurality of doses of medicament can be metered therefrom by abrasion.

12. A tightly compacted body of powdered inhalation medicament according to Claim 11 obtainable by applying a pressure of from 30x10⁴ to 150x10⁴Nm⁻² to a loose powder comprising an active ingredient having a particle size of from 1 to 10µm.

13. A tightly compacted body of powdered inhalation medicament adapted for use in a device as defined in any one of claims 1 to 4 or 6 to 10, the degree of compaction being sufficient to impart the body with structural integrity upon handling.

## Patentansprüche

1. Gerät zur Verabreichung eines Medikaments in Pulverform durch Inhalation, mit einem Medikamentbehälter (3) zur Aufnahme eines Preßkörpers aus pulverförmigem Medikament (10) und einer Dosiereinrichtung (11) zur Abgabe von Medikament aus dem Behälter (3), welche Dosiereinrichtung ein Mittel zum Abreiben des Preßkörpers (10) enthält, dadurch gekennzeichnet, daß ein Feder- oder Schraubmittel vorgesehen ist, um den Medikament-Preßkörper und Abreibmittel aufeinander zu zu bewegen, um zu ermöglichen, daß die richtige Menge Medikament vom Medikament-Preßkörper für eine Dosis abgerieben wird.

2. Gerät zur Verabreichung eines Medikaments in Pulverform durch Inhalation mit einem Medikamentbehälter (3), enthaltend einen Preßkörper aus pulverförmigem Medikament (10), und mit einer Dosiereinrichtung (11) zur Abgabe von Medikament aus dem Behälter (3), welche Dosiereinrichtung ein Mittel zum Abreiben des Preßkörpers (10) enthält, dadurch gekennzeichnet, daß ein Feder- oder Schraubmittel vorgesehen ist, um den Medikament-Preßkörper und Abreibmittel aufeinander zu zu bewegen, um zu ermöglichen, daß die richtige Menge Medikament vom Medikament-Preßkörper für eine Dosis abgerieben wird.

3. Gerät nach Anspruch 1 oder 2, worin das Mittel zum Abreiben ein Messer (11) aufweist.

4. Gerät nach einem der vorhergehenden Ansprüche, worin der Preßkörper (10) durch Federmittel (13) zum Abreibmittel (11) hin gedrückt wird.

5. Gerät nach einem der vorhergehenden Ansprüche, worin der Preßkörper aus pulverförmigem Medikament (10) locker gepreßt ist.

6. Gerät nach einem der Ansprüche 1 bis 4, worin der Preßkörper aus pulverfdrmigem Medikament (10) dicht gepreßt ist, wobei der Preßgrad ausreichend ist, um dem Körper bei der Manipulation strukturelle Integrität zu verleihen.

7. Gerät nach einem der vorhergehenden Ansprüche, worin der aktive Bestandteil des Medikaments hygroskopisch ist.

8. Gerät nach einem der vorhergehenden Ansprüche, worin der aktive Bestandteil des Medikaments ausgewählt ist aus Natriumchromglykat und Nedocromilnatrium.

9. Gerät nach einem der vorhergehenden Ansprüche, worin das Medikament einen inerten Träger enthält.

10. Gerät nach einem der vorhergehenden Ansprüche, worin eine vom Behälter (3) abgegebene bemessene Dosis in einer durchgehenden Bahn abgelegt wird, welche eine Kammer (21) enthält, in der mitgerissenes Medikament zirkulieren kann, wobei der Einlaß (20) von der Bahn zur Kammer (21) tangential zur Kammerwand und normal zur Längsachse der Kammer verläuft und der Auslaß (23) aus der Kammer (21) auf der Längsachse der Kammer liegt, so daß mitgerissenes Medikament vorzugsweise durch den Auslaß (23) aus dem mittleren Bereich der Kammer (21) entfernt wird, wobei die Einlaßbahn (20) benachbart dem Übergang von der Einlaßbahn zur Kammer (21) eine Venturidüse (24) aufweist.

11. Preßkörper aus pulverförmigem Inhalationsmedikament zur Verabreichung aus einem Inhalationsgerät mit einem Medikamentbehälter (3) zur Aufnahme des Preßkörpers aus pulverförmigem Medikament (10) und einer Dosiereinrichtung (11) zur Abgabe von Medikament aus dem Behälter (3), welche Dosiereinrichtung ein Mittel zum Abreiben des Preßkörpers (10) enthält, dadurch gekennzeichnet, daß der Körper ein lockeres Pulver umfaßt, das einen aktiven Bestandteil mit einer Teilchengröße von 1 bis 10 µm enthält, welches zur Bildung eines dicht gepreßten Körpers komprimiert ist, wobei der Preßgrad ausreichend ist, um dem Körper bei der Manipulation strukturelle Integrität zu verleihen, so daß eine Mehrzahl von Dosen Medikament durch Abreiben abdosiert werden kann.

12. Dicht gepreßter Körper aus pulverförmigem Inhalationsmedikament nach Anspruch 11, erhältlich durch Aufbringen eines Drucks von 30x10⁴ bis 150x10⁴ Nm⁻² auf ein lockeres Pulver, welches einen aktiven Bestandteil mit einer Teilchengröße von 1 bis 10 µm umfaßt.

13. Dicht gepreßter Körper aus pulverförmigem Inhalationsmedikament zur Verwendung in einem Gerät nach einem der Ansprüche 1 bis 4 oder 6 bis 10, wobei der Preßgrad ausreichend ist, um dem Körper bei der Manipulation strukturelle Integrität zu verleihen.

## Revendications

1. Dispositif pour l'administration par inhalation d'un médicament sous forme pulvérulente, comprenant un réservoir à médicament (3) propre à recevoir un comprimé d'un médicament pulvérulent (10) et un dispositif de dosage (11) pour dispenser le médicament à partir du réservoir (3), le dispositif de dosage comportant un dispositif pour abraser le comprimé (10), caractérisé en ce qu'un moyen de sollicitation élastique ou à vis est prévu pour déplacer le comprimé de médicament et le dispositif à abraser l'un vers l'autre afin que la quantité de médicament correcte pour une dose puisse être abrasée du comprimé de médicament.

2. Dispositif pour l'administration par inhalation d'un médicament sous forme pulvérulente, comportant un réservoir à médicament (3) contenant un comprimé d'un médicament pulvérulent (10) et un dispositif de dosage (11) pour dispenser le médicament à partir du réservoir (3), le dispositif de dosage comportant un dispositif pour abraser le comprimé (10), caractérisé en ce qu'un moyen de sollicitation élastique ou à vis est prévu pour déplacer le comprimé de médicament et le dispositif à abraser l'un vers l'autre afin que la quantité de médicament correcte pour une dose puisse être abrasée du comprimé de médicament.

3. Dispositif selon l'une quelconque des revendications 1 ou 2, dans lequel le dispositif à abraser comprend une lame (11).

4. Dispositif selon l'une quelconque des revendications précédentes, dans lequel le comprimé (10) est sollicité en direction du dispositif d'abrasion (11) par un moyen de sollicitation élastique (13).

5. Dispositif selon l'une quelconque des revendications précédentes, dans lequel le comprimé de médicament pulvérulent (10) est comprimé lâchement.

6. Dispositif selon l'une quelconque des revendications 1 à 4, dans lequel le comprimé de médicament pulvérulent (10) est comprimé de manière dense, le degré de compression étant suffisant pour conférer une intégrité structurelle au comprimé pendant les manipulations.

7. Dispositif selon l'une quelconque des revendications précédentes, dans lequel le principe actif du médicament est hygroscopique.

8. Dispositif selon l'une quelconque des revendications précédentes, dans lequel le principe actif du médicament est sélectionné dans un groupe comprenant le cromoglycate sodique et le nédocromil sodium.

9. Dispositif selon l'une quelconque des revendications précédentes, dans lequel le médicament contient un véhicule inerte.

10. Dispositif selon l'une quelconque des revendications précédentes, dans lequel une dose mesurée et dispensée à partir du réservoir (3) est déposée dans un conduit de traversée comportant une chambre (21) à l'intérieur de laquelle le médicament entraîné peut circuler, dans lequel le passage d'entrée (20) qui mène du conduit à la chambre (21), est tangentiel à la paroi de la chambre et perpendiculaire à l'axe longitudinal de la chambre, et l'orifice de sortie (23) de la chambre (21) est situé sur l'axe longitudinal de la chambre, de sorte que le médicament entraîné est préférentiellement évacué par l'orifice de sortie (23) à partir de la zone centrale de la chambre (21), le passage d'entrée (20) comportant un venturi (24) adjacent à la jonction du passage d'entrée avec la chambre (21).

11. Comprimé d'un médicament pulvérulent à inhaler pour administration à partir d'un dispositif d'inhalation comportant un réservoir à médicament (3) propre à recevoir ledit comprimé de médicament pulvérulent (10) et un dispositif de dosage (11) pour dispenser le médicament à partir du réservoir (3), le dispositif de dosage comportant un dispositif destiné à abraser le comprimé (10) ; caractérisé en ce qu'il comprend une poudre meuble contenant un principe actif dont les particules ont une granulométrie de 1 à 10 µm, qui a été comprimée pour former un corps comprimé de manière dense, le degré de compression étant suffisant pour conférer au comprimé une intégrité structurelle pendant les manipulations telle qu'il sera possible d'y prélever par abrasion plusieurs doses de médicament.

12. Comprimé dense d'un médicament pulvérulent à inhaler selon la revendication 11, que l'on peut obtenir par application d'une pression de l'ordre de 30 x 10⁴ et 150 x 10⁴ Nm⁻² à une poudre meuble contenant un principe actif dont les particules ont une granulométrie de 1 à 10 µm.

13. Comprimé dense d'un médicament pulvérulent à inhaler propre à être utilisé dans un dispositif tel que défini dans l'une quelconque des revendications 1 à 4 ou 6 à 10, le degré de compression étant suffisant pour conférer une intégrité structurelle au comprimé pendant les manipulations.
